(19) Europäisches Patentamt / European Patent Office / Office européen des brevets

(11) **EP 2 879 565 B1**

## (12) EUROPEAN PATENT SPECIFICATION

(45) Date of publication and mention
of the grant of the patent:
**26.10.2016 Bulletin 2016/43**

(21) Application number: **13745898.0**

(22) Date of filing: **05.08.2013**

(51) Int Cl.:
**A61B 5/00** *(2006.01)*      **G01T 1/22** *(2006.01)*

(86) International application number:
**PCT/GB2013/052087**

(87) International publication number:
**WO 2014/020359 (06.02.2014 Gazette 2014/06)**

(54) **FIBRESCOPE FOR OPTICAL IMAGING OF RADIOPHARMACEUTICALS**

FIBERSKOP ZUR OPTISCHEN BILDGEBUNG VON RADIOPHARMAKA

FIBROSCOPE POUR IMAGERIE OPTIQUE DE PRODUITS RADIOPHARMACEUTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.08.2012 US 201261679449 P
03.08.2012 GB 201213827
12.02.2013 US 201361763884 P**

(43) Date of publication of application:
**10.06.2015 Bulletin 2015/24**

(73) Proprietor: **Lightpoint Medical Ltd
Chesham
Buckinghamshire HP5 1NZ (GB)**

(72) Inventors:
• **TUCH, David
Rickmansworth
Hertfordshire WD3 1RE (GB)**
• **COLLIER, Nicholas
Harston
Cambridgeshire CB22 7GG (GB)**

• **VYAS, Kunal
Harston
Cambridgeshire CB22 7GG (GB)**
• **MORRISON, Euan
Harston
Cambridgeshire CB22 7GG (GB)**

(74) Representative: **Jaeger, Michael David et al
Withers & Rogers LLP
4 More London Riverside
London SE1 2AU (GB)**

(56) References cited:
**US-A1- 2006 281 992     US-A1- 2011 250 128**

• **SRI-RAJASEKHAR KOTHAPALLI ET AL:
"Endoscopic imaging of Cerenkov
luminescence", BIOMEDICAL OPTICS EXPRESS,
vol. 3, no. 6, 3 May 2012 (2012-05-03), pages
1215-1225, XP055080242, DOI:
10.1364/BOE.3.001215 cited in the application**

## Description

### FIELD OF INVENTION

This invention has to do with methods and apparatus for optical imaging of radiopharmaceuticals, in particular Cerenkov Luminescence imaging using a fibrescope.

### BACKGROUND

Robertson et al. (Phys Med Biol. 2009) observed that certain diagnostic radiopharmaceuticals used in nuclear medicine scans can also be imaged optically. Specifically, radiopharmaceuticals that emit charged particles (e.g., alpha and beta particles) generate detectable light due to the phenomenon of Cerenkov luminescence. Cerenkov photons are due to the deceleration of the charged particle in tissue. Optical imaging of charged particle-emitting radiopharmaceuticals is termed Cerenkov Luminescence Imaging (CLI).

CLI combines the advantages of optical imaging (including high spatiotemporal resolution and low cost and form factor) with the advantages of nuclear imaging (including molecular specificity and widespread commercial availability of radiopharmaceuticals). Optical imaging will be understood to include ultraviolet to near infra-red wavelengths.

It would be desirable to use CLI in clinical situations, for example to provide images to inform a surgeon during the course of a procedure. One technical challenge for performing CLI in a clinical scenario is that the Cerenkov spectrum for commonly used diagnostic radioisotopes (particularly those labeled with Fluorine-18) is in the visible spectrum between 400-800 nm. The background illumination in the room would interfere and dominate the Cerenkov spectrum. Also, the illumination would induce tissue auto-fluorescence in the visible spectrum which would overlap with the Cerenkov signal.

Consequently, CLI is not feasible under typical lighting conditions. All of the CLI applications to date have been in pitch black rooms or chambers where there is no light interference. CLI methods and systems are described in: US 2011/0250128; Holland et al., Mol Imaging, 2011; Carpenter et al., J Nucl. Med, 2012; US2012/0220870; and Kothapalli et al., Biomedical Optics Express, Vol 3, No 6, 1 June 2012.

It has also been proposed to use CLI to create 3D images by means of tomography, as described in WO 2012/083503 and in Zhong et al, International Journal of Biomedical Imaging, Vol 2011, Article ID 641618. WO 2011/137247 also describes a method of 3D imaging of Cerenkov luminescence, based on the intensity profile of the Cerenkov light. However, these methods also require pitch-black conditions and this are infeasible in a clinical setting.

The journal article "Endoscopic imaging of Cerenkov Luminescence" (Biomedical Express vol. 3 no. 6, 3 May 2012) is directed to a feasibility study of an endoscopic imaging system capable of imaging Cerenkov light emitted from radionuclides.

### SUMMARY OF INVENTION

The present invention is concerned with methods and apparatus for optical imaging of radiopharmaceuticals that are practical for clinical settings, for example, in an operating theatre.

In particular, the inventors have identified a need for imaging tissue in situ at an open surgical site or other clinical site on a patient. For example, during a procedure to remove abnormal (e.g. cancerous) tissue from a subject it would be very beneficial for a surgeon to be able to confirm prior to conclusion of the procedure that they have removed all of the abnormal tissue; a common problem in such procedures is that a marginal portion of the abnormal tissue is left behind, which must be removed in a further procedure once it is later detected. A proposal of the present invention is therefore to provide a imaging system and method that use Cerenkov luminescence imaging to inspect the tissue at a clinical site such as an open surgical site to check that the all abnormal tissue (e.g. cancerous cells) has been removed.

In a first aspect, the invention provides an apparatus configured for optical imaging of Cerenkov luminescence from a region on a subject subsequent to the subject receiving a dose of a radiopharmaceutical, the apparatus comprising:

   an imaging means capable of imaging Cerenkov photons;
   a fibrescope for transmitting light received at a distal end of the fibrescope to a proximal end of the fibrescope, the proximal end of the fibrescope being connected to the imaging means; and
   an optical shroud surrounding the distal end of the fibrescope and, when in use, covering a region of interest, wherein the optical shroud is capable of at least substantially blocking the passage of ambient light.

The optical shroud is capable of at least substantially (and preferably completely) blocking the passage of ambient light. In some embodiments the shroud is designed to attenuate ambient light by at least 10 to 14 orders of magnitude.

It is preferred that the ambient light level received by the imaging means should result in a photon flux less than 10 times the photon flux from the radiopharmaceutical, otherwise it may be very difficult or impossible to see the Cerenkov image. More preferably the photon flux resulting from ambient light is no more than 10 times less than the radiopharmaceutical flux. The flux from the radiopharmaceutical (e.g. F18) will typically be between $10^3$ and $10^4$ photons/s/sr/cm$^2$.

In this way, when the shroud is place over a region of interest (for example, a surgical site), with a bottom edge of the shroud forms a seal against the subject's skin around the region of interest (either directly or using additional sealing parts, for example as discussed below), a light tight enclosure is formed to enable Cerenkov imaging of the region of interest.

The shroud may be formed of any of a number of suitable materials or combinations of materials that prevent penetration of ambient light to an adequate degree to enable successful Cerenkov imaging. It may for example be a rubberised black-out fabric, a rubber sponge material such as closed cell expanded neoprene, metalised films, opaque moulded polymers.

In some embodiments, to help ensure a light tight seal of the shroud against the subject, an opaque drape is first placed over the subject, the drape having an opening such that it does not cover the region of interest. A bottom edge of the shroud can then be brought into contact with the drape around the region of interest.

Rather than rely on the shroud sitting tightly against the drape, it is preferred that sealing means are provided to secure the shroud to the drape. The sealing means may, for example, be physical (e.g. Velcro™), magnetic, vacuum or electrostatic seals. Alternatively the shroud and seal may be physically joined to one another by a connector, such as a rigid ring that is engaged by the shroud and the drape. Another alternative is to engage the seal using an external power source, such as a vacuum line.

In some embodiments there is a light sensor within the shroud that can be used to confirm whether or not a light tight enclosure has been created. In other embodiments, images collected by the imaging means can be used for this confirmation (especially where the imaging means is configurable to collect illuminated images, as discussed below).

The imaging means may be a charge coupled device (CCD) camera. A cooled electron-multiplying CCD (emCCD) camera is preferred to acquire the low light level CLI images. Possible alternative imaging means include intensified CCD, photon multiplier tube (PMT) array, or micro-channel plates with electron collection by one or more electrodes.

When using an emCCD camera to image Cerenkov photons, the EM gain will typically be set to at least 100, preferably at least 200 and more preferably to about 300. Higher EM gains may be used. For example, for photon counting a gain of as much as 1000 might be used. When acquiring the Cerenkov images the emCCD camera will be cooled, typically to -80 to -100 degrees C.

In some embodiments, the Cerenkov imaging means (e.g. emCCD camera) is housed within a radiation shield to help avoid interference from unwanted radiation, such as gamma rays, or beta particles. Suitable forms of shielding include lead shielding and Boron-filled high density polyethylene for example. Other materials or composite structures that can block the unwanted radiation can also be used.

The fibrescope comprises a lens mounted on the distal end of a light conduit for conveying an optical signal from the lens to the imaging means. The light conduit will typically be a coherent fibre optic bundle, with the lens mounted on its distal end. The fibrescope may have a conventional construction and will normally be flexible to allow it to be easily manipulated by the operator. The diameter of the lens may be about 1.5cm. In some embodiments, the focus of the lens can be varied. Preferably the adjustment of the lens focus is motorised so that controls can be provided outside the shroud to facilitate focusing the lens when the shroud is in position over a region of interest.

In some embodiments, the fibre optic bundle within the fibrescope (or the complete fibrescope) is shielded by a radiation shield. Similarly to the shield for the imaging means, this is to help avoid interference from unwanted radiation, such as gamma rays, or beta particles in the fibreglass of the fibrescope. Suitable flexible radiation-shielding materials include, for example, metal-impregnated elastomers.

In many cases it will be useful for the operator of the apparatus to be able to view an illuminated image (e.g. a white light image) of the region of interest within the light-tight enclosure formed by the shroud before and/or during acquisition of Cerenkov images.

Prior to acquisition of the Cerenkov image this may be useful, for example, to ensure correct positioning of the fibrescope and correct focus of the lens. During acquisition it may be useful in order to monitor movement of the fibrescope relative to the region of interest and/or to provide sequences of illuminated and Cerenkov images that can be overlaid upon one another e.g. to ensure correct registration of sequential Cerenkov images. This registration of images may be particularly important in some embodiments, where for example the fibrescope is held by a person who is liable to move. The Cerenkov image is typically averaged from multiple frames and if the subsequent frames are not spatially registered with one another then there will be a loss of resolution. Any change in position of the fibrescope relative to the region of interest can be identified in the illuminated image and the registration of the Cerenkov frames corrected accordingly with suitable image processing procedures (e.g. using image processing software).

In some cases, a surgeon may also want an illuminated image in order that they can conduct a procedure (e.g. excision of tissue) using instruments within the shroud. Particularly in the cases of using an illuminated image pre-Cerenkov acquisition to ensure correct location of the fibrescope, or to give a surgeon visibility of the region of interest to conduct a procedure, the image may conveniently be a video image.

In order to obtain an illuminated image from within the shroud it is necessary to illuminate the interior of the light tight enclosure formed by the shroud. The preferred approach to illuminate the enclosure is to employ a separate illumination channel in the fibrescope through which light from an illuminated source (e.g. white light source or R/G/B light source, which may be one or more LEDs) can be transmitted from the proximal end to the distal end of the fibrescope to provide an illumination source at the tip of the fibrescope within the shroud. Alternatively, one of more LEDs may be mounted within the shroud, for example on the distal end of the fibrescope.

It is envisaged that an operator will switch the apparatus between illuminated and Cerenkov imaging modes. Some embodiments may include a third mode for simultaneous illuminated image capture and CLI.

In embodiments where multiple illuminated and Cerenkov images are to be acquired alternately with one another (i.e. time multiplexed) it is necessary to "switch off" the white/RGB lights during periods of CLI acquisition. In preference to actually switching off the lights (e.g. LEDs), a mechanical shutter may be used to cover the light source(s) during periods of CLI acquisition and to uncover the light source(s) during periods of illuminated image acquisition. This approach has the advantage of near instantaneous "switching off" of the lights, avoiding problems that might otherwise result from the period of time it takes for a light to cease emitting once it is no longer powered.

The mechanical shutter will be synchronised to the image acquisition sequence. The shutter may, for example, be a rotating disc with regularly spaced cut outs round its circumference so that the disc can intermittently cover and uncover the light source(s). The rotation of the disc can then be controlled to give the desired periods of light and dark within the shroud, with which the illuminated and Cerenkov image acquisitions can be synchronised. Where separate cameras are used for illuminated image acquisition and CLI respectively, a mechanical shutter may also be used for the CLI imaging means (to avoid damage to the imaging means during periods of illuminated image acquisition). The shutter for the CLI imaging means may also be a rotating disc. Conveniently, the CLI imaging means and the light source for illuminating the chamber can be arranged so that a single rotating disc can serve as a shutter for the imaging means and the light source.

During such time multiplexed illuminated image and CLI acquisition, typically the duration on CLI acquisition periods will be longer than illuminated image acquisition periods to allow time to capture an adequate Cerenkov image, given the much lower light intensity levels. The CLI acquisition period may be many times as long as the illuminated periods, for instance about 3 to 20 times as long. For example, for a 10Hz illuminated video rate, the illuminated period would take 17 ms, with 75 ms of CLI acquisition in-between frames. 8 ms are lost every cycle due to the shutter transition times. The CLI imaging is only enabled whilst the light shutter is firmly closed and not in transition.

In some embodiments the CLI acquisition does not commence immediately after the light is 'switched off' (e.g. covered by the shutter) to give time for any residual light in the chamber to disperse.

In some embodiments it may be desirable to employ two separate imaging means, one to capture the illuminated image and the other to capture the CLI image. In other embodiments, a single imaging means captures both images.

In the case where a single imaging means is used it will normally be desirable to switch the imaging means between an illuminated level image mode and a low light level image mode for capture of the illuminated and CLI images respectively. This is because the types of camera that are sensitive enough to detect Cerenkov photons are likely to be damaged if the same sensitivity settings are used for an illuminated image. Where a single camera is used, the level of light for the illuminated image is preferably low and may be achieved, for example, by using neutral density filters and pulse width modulation of LEDs.

For example, in the case of an emCCD camera, whilst it will be cooled significantly and the EM gain set to a relatively high level for CLI, the camera will typically be operated in a conventional CCD mode, with no EM gain when capturing illuminated images. Furthermore, in order to avoid ghosting it would be necessary to raise the temperature of the sensor to ambient before cooling it down once more for the next CLI image.

In some embodiments of the invention, therefore, the imaging means is switched between operating modes (Cerenkov image capture and illuminated image capture modes) during operation, this switching being synchronised with the switching on and off (e.g. by the mechanical shutter) of the light(s) in the chamber as the apparatus switches back and forth between Cerenkov image capture and illuminated image capture.

Particularly where the apparatus is to be used during a procedure for removing abnormal tissue, examination of the margins, especially the surface of the open region of interest can be important (as noted above). The inventors have recognised that there the sensitivity is reduced by the charged particles at the surface that escape the tissue and therefore cease generating Cerenkov photons. The sensivity of CLI can be increased by placing a Cerenkov radiator on the surface of the tissue so that the escaping charged particles generate Cerenkov light. The Cerenkov radiator should have high refractive index (1.5<RI<2.4), high transmission at short wavelengths (<500 nm), and sufficiently thin to minimize scattering of the charged particles.

In some embodiments, therefore, it is proposed to use a Cerenkov radiator consisting of a cover slip or mesh with the properties indicated above. The Cernekov radiator can be placed over the tissue surface at the region of interest.

The interaction with the cover slip or mesh of charged particles from the tissue surface generates Cerenkov photons and/or scintillation photons that can then be imaged by the imaging means.

Suitable high refractive index materials for the cover slip or mesh include lead glass, zirconium glass, or tellurite glass. A mesh is advantageous because it can better conform to an irregular-shape tissue surface. The mesh may be formed, for example, using discrete sections of high refractive index material held together by a flexible mesh lattice, for example of polyurethane.

In some embodiments, to maintain sterility, the fibrescope is housed within a sterile casing. The casing may be connected to the shroud. In one embodiment the sterile casing is made of a flexible material (e.g., silicone) with a transparent window (for example, made of glass) on the distal end. In another embodiment, the sterile casing consists of a hard material (for example, metal) with a transparent window on the distal end, and the casing is sterilisable (for example, by autoclave or ethylene oxide gas).

In a second aspect, the invention provides a method for optical imaging of Cerenkov luminescence from a region of interest on an object subsequent to the object receiving a dose of a radiopharmaceutical, the apparatus comprising:

covering the region of interest with a shroud to form a light tight enclosure over the region of interest; and
capturing a Cerenkov luminescence image of the region of interest using a fibrescope having a distal end within the shroud and a proximal end outside of the shroud connected to an imaging means.

In some embodiments, the method further comprises illuminating the interior of the shroud with light and capturing an illuminated image of the region of interest whilst the interior of the enclosure is illuminated, the Cerenkov luminescence image being captured when the interior of the enclosure is not illuminated.

The method may employ the apparatus of the first aspect above, including any one or more of its preferred and optional features discussed above.

In some embodiments the object is a subject such as a patient undergoing a clinical (e.g. surgical) procedure.

In other embodiments, the object is a sample or specimen. In this case the shroud may be a flexible light tight shield that is held over the sample, for example on a table top or a tray (e.g. a surgical tray).

Alternatively, the shroud may be a light tight container such as a sample dish with a lid having opaque walls to exclude ambient light from the inside of the container. Conveniently, the container may be disposable. The distal end of the fibrescope may be exposed to the interior of the container for example through a sealed opening in a wall of the container or the container lid.

A method and apparatus, not forming part of the present invention, are also described, for optical imaging of Cerenkov luminescence from a subject subsequent to the subject receiving a dose of a radiopharmaceutical, the method comprising the steps of:

capturing a first image of the subject;
capturing a second image of Cerenkov luminescence; and superimposing the two images.

Preferably the first and second images may comprise a sequence of images (e.g. a video).

Preferably the steps of capturing the first image and the second image are repeated rapidly in sequence, or more preferably simultaneously, so that a video of the subject formed from the overlaid or superimposed images is created. The video rate of the first and second images may vary significantly, for example with the CLI image being semi-static at 0.2 Hz, and the white light image being dynamic at 10Hz.

A method, not forming part of the present invention, is also described for optical imaging of Cerenkov luminescence from a subject subsequent to the subject receiving a dose of a radiopharmaceutical, the method comprising the steps of:

illuminating the subject with light of a predetermined range of wavelengths; capturing a first image with a first imaging means; and capturing a second image with a second imaging means, said second image captured at wavelengths distinct from that of the illuminating light.

Preferably, the methods of the invention include placing an optical shroud or opaque drape to shield the region to be imaged. In this way, it is not necessary to interfere with the lighting in an operating theatre.

Preferably, the method includes use of a light detector and indicator to determine the light level within the shroud or drape. This makes it possible to check that the surgical site is sufficiently shrouded to perform CLI.

Preferably, the method includes the step of applying strobed or spectrally separated lighting. Preferably, the strobed or spectrally separated lighting may be applied within the optical shroud or drape. The strobe or spectrally separated lighting may be applied by a light source within the room.

An apparatus, not forming part of the present invention, is also described for optical imaging of Cerenkov luminescence from a subject subsequent to the subject receiving a dose of a radiopharmaceutical, the apparatus comprising a first imaging means for capturing a first image of the subject, and a second imaging means for capturing a second image of the subject, wherein said second image is captured at wavelengths of light distinct from that of the illuminating light.

Preferably, the first imaging means images the subject over wavelengths ranging from the ultra-violet to visible to nfra-red.

Preferably the two images from the first and second imaging means are superimposed to produce a single image for use e.g. by a surgeon performing surgery to remove cancerous tissue that has taken up the radiopharmaceutical.

Again, preferably the steps of capturing the first image and the second image are repeated rapidly in sequence, or more preferably simultaneously, so that a video of the subject formed from the overlaid or superimposed images is created.

Preferably, the subject is illuminated only with light at the red end of the spectrum e.g. from monochromatic red or monochromatic blue LED lighting. Preferably, the subject is illuminated only with light in the range of 500-740 nm, preferably in the range 625-740 nm, or in the range 435-500 nm. The skilled person will appreciate that other predetermined ranges of wavelengths of light may be used to illuminate the subject, e.g. red light, or non-contiguous ranges of light (e.g. red light and blue light).

It is beneficial for the subject to be illuminated to allow e.g. a surgeon to be able to operate on the subject.

Red illumination may be preferable to blue illumination since green-to-violet light induces tissue autofluorescence in the visible range.

Preferably the second imaging means images Cerenkov luminescence to perform CLI. Preferably the second imaging means is ultra-sensitive and is optimised to perform CLI. For example, preferably the second imaging means is cooled to eliminate background noise (commonly called "dark noise"). Preferably the first and second images are calibrated.

Preferably the light paths feeding the two imaging means pass through a beam-splitter, so that they both image the same region of the subject. The beam-splitter is preferably a dichroic prism.

Preferably the second imaging means has a band pass filter to block any residual light and to aid in selecting the discrete and distinct wavelengths of light to be imaged. The need for the band pass filter may depend on the performance of the beam-splitter. The light source may also use a filter to further minimize spectral overlap.

It will be understood by the skilled person that the first and second imaging means can be contained within the same apparatus. Preferably the imaging means is a camera. It will be understood by the skilled person that the imaging means could be a charge-coupled apparatus (CCD) contained within a camera. The first imaging means and the second imaging means may be two different CCDs contained within the same unit. Using two imaging means is preferable because it allows for the spectral response and dynamic range to be selected separately for each image. In addition, strong illumination of a sensitive camera increases the dark noise for a period after illumination. The CCD for the Cerenkov camera could have high quantum efficiency in the near infra-red range. The chip could comprise, for example, an electron multiplying CCD, intensified CCD, photon multiplier tube (PMT) array, or micro-channel plates with electron collection by one or more electrodes.

Preferably the second imaging means is also enclosed within a radiation shield (e.g. lead shielding or Boron-filled high density polyethylene) to block any interference from unwanted radiation, such as gamma rays, or beta particles.

Preferably the plane of the imaging means (e.g. CCD) within the second imaging means may also be placed parallel to the incoming light to minimize the cross-section exposed to unwanted radiation, such as gamma rays, or beta particles.

Preferably image processing is applied to the two images obtained from the first and second imaging means to calibrate the intensity windowing and apply image registration if required. To further segment the image from the second imaging means, additional imaging processing may be performed on this image including both spectral and spatial information. For example, it can be specified that the image from the second imaging means only comes from a restricted field-of-view (e.g. the surgical site) within the image. Another example is that the signal within a pixel should fit the expected spectrum emitted from the radiopharmaceutical (e.g. the Cerenkov spectrum).

Preferably, an optical shroud or opaque drape is placed to shield the region to be imaged. In this way, it is not necessary to interfere with the lighting in an operating theatre, which is advantageous in case of emergencies.

Preferably, a light detector and indicator may be used to check that the region to be imaged (e.g. clinical site) is sufficiently shrouded to perform CLI.

A method, not forming part of the present invention, is also described for optical imaging of Cerenkov luminescence from a subject subsequent to the subject receiving a dose of a radiopharmaceutical, the method comprising the steps of: illuminating the subject with light from a light source;

capturing a first image whilst the subject is illuminated with light from the light source; and

capturing a second image whilst the subject is not illuminated with light from the light source.

Preferably the region of interest is illuminated with light from the light source for the majority of the time. Preferably the light from the light source is emitted in repeated pulses. Preferably strobe illumination should be >100 Hz and the pulses should be 10-1000 microseconds in duration. Strobe illumination at >100 Hz is typically imperceptible e.g. by a surgeon performing on the subject. Whilst the subject is illuminated with this light from the light source, a first image, or series of first images, is obtained of the subject. Then, for short intervals between the pulses of illumination, the light source is switched off so that the subject is not illuminated with light from the light source. Preferably during this interval, the subject is in the dark. During this interval, the second image, or series of second images, of the subject may be obtained. The second image may detect Cerenkov luminescence during this interval. This sequence may be repeated resulting in repetitions or pulses of imaging with light from the light source and imaging Cerenkov luminescence with no light from the light source. The first and second images or sequences of images are then preferably overlaid or superimposed. Preferably a video of sequences of overlaid or superimposed first and second images is produced.

An apparatus, not forming part of the present invention, is also described for optical imaging of Cerenkov luminescence from a subject subsequent to the subject receiving a dose of a radiopharmaceutical, the apparatus comprising a first imaging means for capturing a first image of the subject, and a second imaging means for capturing a second image of the subject, wherein the first and second imaging means are connected to a stroboscopic illuminating apparatus, and said second image is captured when the subject is not illuminated by the stroboscopic illuminating apparatus.

Preferably the first image is obtained with a first imaging means and the second image obtained with a second imaging means. However it will be understood that the first and second images may be obtained with the same imaging means. This is important as it may reduce costs, as imaging means suitable for these applications can be expensive.

Preferably the time offset or gating offset between the strobe pulse and the acquisition of the second image is sufficiently long to allow for the decay of any induced tissue autofluorescence, and also for any charge on the imaging means (e.g. CCD) to be cleared.

Preferably the acquisition of the second image is gated off of a signal from the illumination system. The gated acquisition can be performed using, for example, a digital micro-mirror apparatus (DMD), a liquid crystal shutter or a spatial light modulator.

Preferably the subject is illuminated by an automatic stroboscopic illumination. More preferably, the automatic stroboscopic illumination is white-light illumination with a gated shutter, for example, using a Pockels cell or digital micro-mirror apparatus (DMD).

Preferably the first image is a structural image and the second image is a Cerenkov image. In cases where the structural and Cerenkov images are measured by the same imaging means, the gated acquisition can be performed by treating segments of the signal as the Cerenkov image.

Preferably the plane of the imaging means (e.g. CCD) may be placed parallel to the incoming light to minimize the cross-section exposed to other sources of radiation such as gamma rays or beta-particles.

Preferably the first and second images are calibrated. Preferably image processing is applied to the first and second images to calibrate the intensity windowing and apply image registration, if required. To further segment the image, additional imaging processing may be performed on this image including both spectral and spatial information. For example, it can be specified that the signal within a pixel should fit the expected spectrum emitted from the radiopharmaceutical (e.g. the Cerenkov spectrum).

If two imaging means are used, preferably the second imaging means is ultra-sensitive and is optimised to perform CLI. For example, preferably the second imaging means is a cooled, electron-multiplying CCD camera.

Preferably the light paths feeding the two imaging means pass through a beam-splitter, so that they both image the same region of the subject. The beam-splitter is preferably a dichroic prism.

Preferably the second imaging means has a band pass filter to block any residual light and to aid in selecting the discrete and distinct wavelengths of light to be imaged. The need for the band pass filter may depend on the performance of the beam-splitter.

It will be understood by the skilled person that the first and second imaging means can be contained within the same apparatus. Preferably the imaging means is a camera. It will be understood by the skilled person that the imaging means could be a charge-coupled apparatus (CCD) contained within a camera. The first imaging means and the second imaging means may be two different CCDs contained within the same unit. Using two imaging means is preferable because it allows for the spectral response and dynamic range to be selected separately for each image. The CCD for the Cerenkov camera could have high quantum efficiency in the near infrared range. The chip could comprise, for example, an electron multiplying CCD, intensified CCD, PMT array, or micro-channel plates with electron collection by one or more electrodes.

Preferably the second imaging means is also enclosed within a radiation shield (e.g. lead shielding or Boron-filled high density polyethylene) to block any interference from gamma rays, or beta-particles.

Preferably the above aspects are such that they could also be applied in other diagnostic imaging procedures such as endoscopy, capsule endoscopy, imaging for robotic surgery, whole-body imaging, and basic research.

A phantom, or testing replica, not forming part of the present invention, for ultra-weak light is described. The phantom may use a light emitting diode (LED) with one or more, for example a stack or layers of, neutral density filters through which light from the LED is emitted. Alternatively or additionally, a wavelength selective attenuator may be used in the LED. Reduction of intensity may alternatively or additionally be achieved using pulse width modulation, which may be varied using electronics or software techniques. The phantom may be used to calibrate the light system, and may also or alternatively be useful for maintenance and/or quality control.

BRIEF DESCRIPTION OF FIGURES

Examples are now described with reference to the accompanying drawings, in which:

Fig. 1 shows an example embodiment of the invention;

Fig. 2 shows an example embodiment of the invention that uses stroboscopic illumination;

Fig. 3 shows an example sequence of stroboscopic illumination, Cerenkov image acquisition and illuminated structural image (e.g. anatomical image) acquisition of an example embodiment of the invention;

Fig. 4a schematically shows an example embodiment of the first aspect of the invention using a fiberscope for Cerenkov luminescence imaging;

Figs. 4b to 4d show variations of the shroud arrangement for the fibrescope embodiment of fig. 4a.

Fig. 5 schematically shows an alternative embodiment that employs two cameras and an optical coupler and shutter to direct light to the two cameras;

Figs. 6a and 6b show two alternative arrangements for the optical coupler used in the embodiment of fig. 5;

Fig. 7a shows a plan view and fig. 7b a cross-section of a rotating disc shutter that can be used to shutter a light source and an emCCD camera.

Fig. 8 is a schematic view of an optical light shield and camera setup used in experiments to illustrate the principles of embodiments of the invention;

Fig. 9 shows the layout of sample wells used in the experiments;

Figs. 10a and 10b show Cerenkov images captured during the experiments; and

Fig. 11 is a graph of signal photon rate for each of the experimental wells.

DETAILED DESCRIPTION

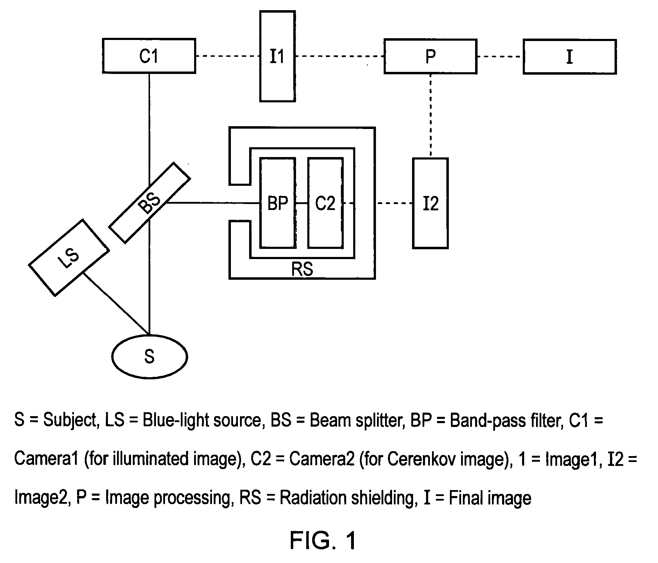

An example embodiment of the invention is shown in Fig. 1. This example embodiment allows CLI to be performed under lit conditions. In this example embodiment, the background lighting is completely eliminated, and monochromatic red LED lighting is used to illuminate the subject. The exemplary set-up of the camera and other components of the imaging system may also be used for embodiments with regular ambient lighting where the part of the subject being imaged is shrouded to exclude the ambient light, as discussed further below.

In this example embodiment, a subject is injected with $^{18}$F-Fluorodeoxyglucose (FDG) (a common beta-emitting radiopharmaceutical). The radiopharmaceutical may be injected systemically, or locally. Commonly, there is a narrow time window of around 60 minutes to 3 hours post-injection for a scan to be performed. This is a result of the pharmacokinetics and radioactive decay of the radiopharmaceutical at the region of interest.

Two separate cameras (C1 and C2) are used to image the illuminated image and the Cerenkov image respectively. Using two separate cameras allows for the spectral response and dynamic range to be selected separately for each image. The second camera (C2) is an ultra-sensitive camera such as a cooled emCCD camera.. For the first camera (C1), one or more monochromatic or colour cameras may be used. By rapidly applying (in any order) sequential red, green and blue illumination and then composing the image, full colour imaging can be provided. The speed at which the illumination is applied is determined by the desired frame rate of the video image.

In alternative embodiments, use of very low levels of illumination and a single camera may be used to take advantage of the sensitivity of the CLI camera. This illumination could be flashed red, green and blue if a colour image is required.

A large aperture lens with low f number is preferred. This arrangement means that more light can be collected. Usually this is undesirable because it leads to distortions. However, the spatial resolution is sufficiently maintained for CLI, which generally has a comparatively poor spatial resolution, so that the improvement in light input outweighs the loss of spatial resolution.

The light generated by the radiopharmaceuticals is passed through a beam splitter (BS) such as a dichroic prism that directs the red light to the first camera and the non-red light to the second camera. The second camera is also equipped with a band-pass filter (BP) to block any residual red light. The need for the band-pass filter will depend on the performance of the beam-splitter. The role of red and blue may be reversed to allow, for example, a surgeon to see deeper into tissue.to direct a portion of the light to the illuminated camera and a portion of the light to the Cerenkov camera.

C2 is also enclosed within a radiation shield (e.g., lead shielding) (RS) to block any interference from gamma rays or beta-particles. The plane of the camera chip within C2 may also be placed parallel to the incoming light to minimize the cross-section exposed to gamma rays or beta-particles.

Image processing (P) is applied to the two images (I1 and I2) to calibrate the intensity windowing and apply image registration, if required. To further segment the Cerenkov image, additional imaging processing can be performed on I2 including both spectral and spatial information. For example, it can be specified that the Cerenkov image only comes from a restricted field-of-view (such as the tumour within the surgical site) within I2. Another example is that the signal within a pixel should fit the expected Cerenkov spectrum. The final image (I) is generated by superimposing the calibrated Cerenkov image (I2) on the illuminated image (I1).

In another embodiment of the invention, CLI can be performed in intervals between stroboscopic pulses of light. In this example embodiment, the subject is illuminated by automatic stroboscopic illumination. In this embodiment the illumination is white-light illumination with a gated shutter using a digital micro-mirror apparatus (DMD). Other methods of shuttering are contemplated within the scope of the invention. In some embodiments, the stroboscopic or spectrally separated, lighting may be provided within an optical shroud. In some embodiments, the stroboscopic or spectrally separated, lighting may be provided in the room.

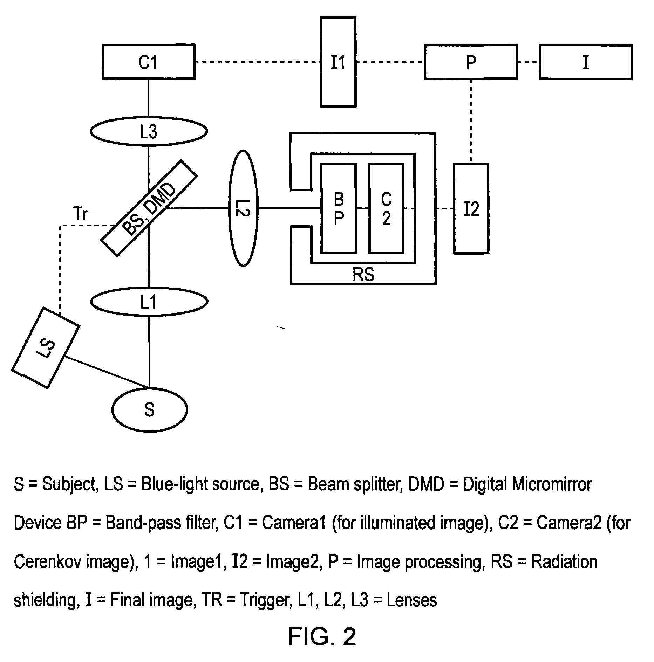

This embodiment uses a similar apparatus setup to the embodiment described above. In this example embodiment the acquisition of the second image is gated off of a signal from the stroboscopic illumination system, as shown by Fig. 2. The gated acquisition is performed using a digital micro-mirror apparatus (DMD). In this example where stroboscopic illumination is used, a trigger (TR) connects the DMD to the light source. Connecting the light source and the DMD by the trigger allows light to be directed to one of two cameras for separate imaging of the Cerenkov or structural images.

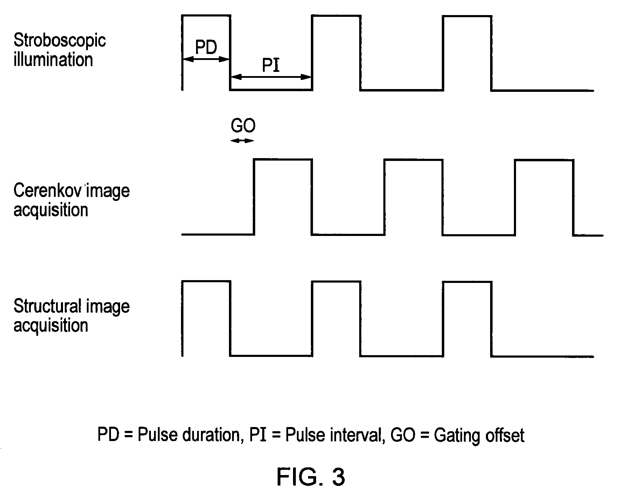

Fig. 3 shows an example sequence of the stroboscopic pulses and intervals. In an example embodiment, strobe illumination may be >100 Hz and the pulse duration (PD) may be 10-1000 microseconds in an example embodiment. Structural image acquisition is performed during the stroboscopic pulse duration. In this example embodiment the time or gating offset (GO) between the strobe pulse and the acquisition of the second image is sufficiently long to allow for the decay of any induced tissue autofluorescence, and also for any charge on the CCD of the camera to be cleared. In an example embodiment, if the pulse duration (PD) is 1000 microseconds, the pulse interval (PI) is 9000 microseconds, and so the gating offset (GO) may be 2000 microseconds and the second (Cerenkov) image acquisition time is 7000 microseconds. In another example embodiment, if the pulse duration (PD) is 10 microseconds, the pulse interval (PI) is 9990 microseconds and so the gating offset (GO) may be 1990 microseconds and the second (Cerenkov) image acquisition time is 8000 microseconds.

In some embodiments, an optical shroud may be used to shield the patient, or the region to be imaged. An optical shroud is a shroud or shield that is capable of substantially preventing the penetration of ambient light through the shroud material, so that CLI measurements are possible without turning off room lights. Optionally, the optical shroud is capable of conforming to desirable shapes. Optionally, the optical shroud is made of a material capable of conforming, for example, to the contours of a patient's body. The shroud may comprise fabric or metal foil. Other materials that achieve the desired effects are contemplated. The shroud may make an optically tight seal with the surface of the patient. An optically tight seal may be achieved using, for example, a gel. The gel may be placed around the rim of the shroud that comes into contact with the patient. The seal should reduce in wavelength "passband" light levels inside the shroud to a level comparable to, or lower than, the CLI signal.

Optionally, the shroud may include built-in gloves to allow, for example, a surgeon access to the region of interest during imaging without breaking the optically tight seal. Alternatively or additionally, instrument ports and/or instruments may be included.

Alternatively or additionally, an image display may be located on a camera.

In some embodiments, a flexible fiberscope or endoscope may be used for Cerenkov luminescence imaging.

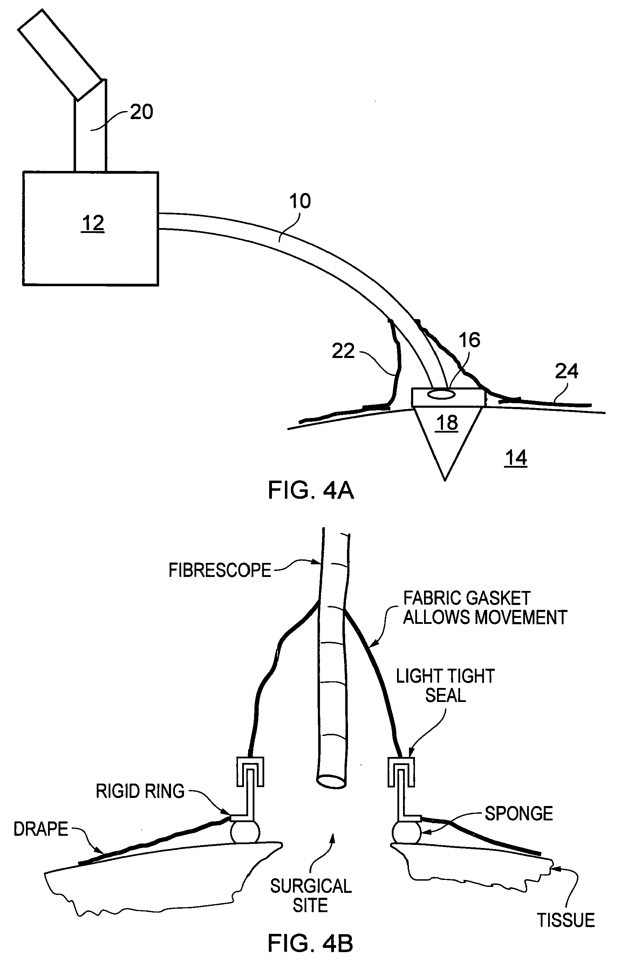

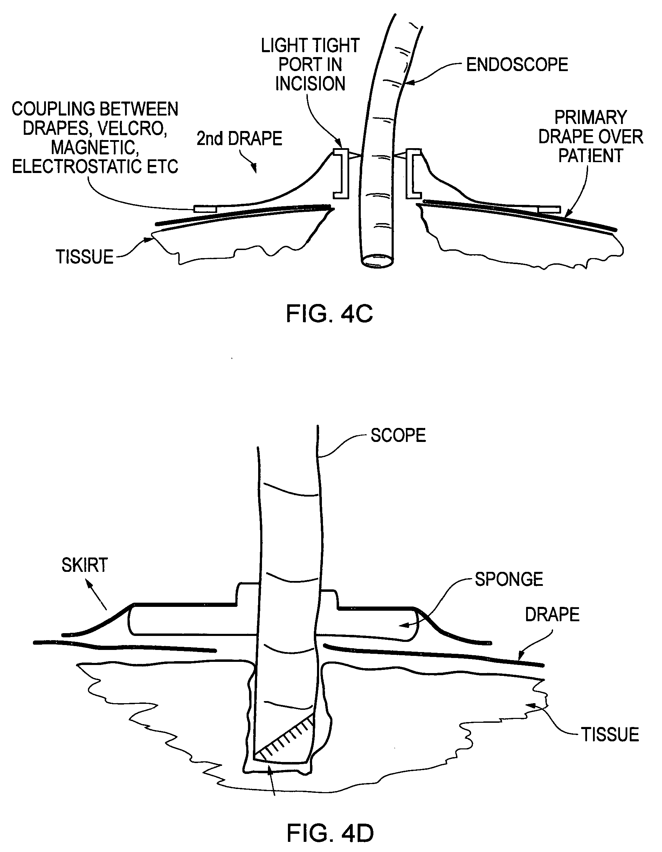

Fig. 4 schematically shows an embodiment in accordance with the first aspect of the invention in which a fibrescope 10 is used to image tissue at a region of interest 18 (for example a surgical site) during a procedure being carried out on a patient 14.

As shown in Fig. 4, the fiberscope 10, which is preferably flexible for a surgeon's ease of use, extends from an electron multiplying charge coupled device camera (emCCD) 12 to the region of interest 18 on the patient 14. It is envisaged that the fibrescope 10 will be held in place by the surgeon or an assistant but in other examples a physical support may be provided. The tip 16 of the fiberscope 10 is used for imaging tissue at the region of interest 18.

The emCCD camera is supported by a boom 20.

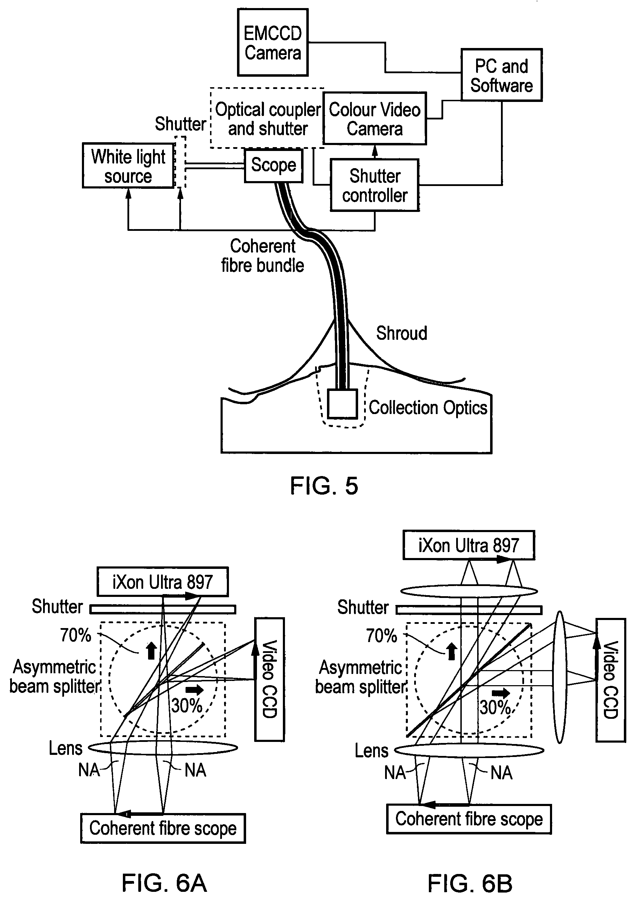

An optical shroud 22 (or opaque drape) surrounds the distal end 16 of the fibrescope 10 and the region of interest 18 so that little or no external light is present inside the optical shroud 22 and external light is prevented from entering the region of interest 18. Preferably, the optical shroud 22 is mounted on the distal end of the fiberscope 10. Sealing of the optical shroud 22 may be provided in the same way as described above for sealing against a patient, for example. Alternative approaches to achieving a light tight seal around the edges of the shroud are discussed below with reference to figs. 5b to 5d. The shroud is also sealed tightly against the fibrescope.

Optionally, a skirt or drape 24 may be used to extend over the patient 14 away from the region of interest 18. The skirt 24 may be attached to the shroud 22 to help block light from entering the region of interest 18.

For example, as seen in fig. 4b, the drape may be attached to a rigid ring and the lower edge of the shroud formed with clasps that can engage a top edge of the ring to form a light tight seal. A sponge member below the ring prevents the ring from pressing into the patient's skin and also help to ensure a light tight seal against the skin is maintained.

Figs. 4c and 4d show alternative arrangements for sealing the shroud to the drape. In fig. 4c, the shroud is connected to the top surface of the drape by a seal that may, for example, be a hook and loop seal, a magnetic seal or an electrostatic seal. In this example, rather than the fibrescope extends into the shroud through a light tight port.

In the example shown in fig. 4d, the seal is created without any physical connection and relies instead on a long overlap between a skirt of the shroud and the drape. In this example a sponge member is also used to enhance the seal.

The tip 16 of the fiberscope 10 may be manipulated by, for example, one or more gloves through the optical shroud 22, by a wire through the optical shroud 22, or by having a rigid end part of the fiberscope 10 near the tip 16 so that it is possible to manipulate the tip 16 from outside the shroud 22. Other ways of manipulating the tip 16 of the fiberscope 10 will be apparent to the skilled person.

It is commonly difficult to tell whether an optically tight seal has formed by eye. Accordingly, in some embodiments a light detector and indicator may be used to provide feedback to, for example, a surgeon, to provide information about the light tightness of the optical shroud. In some embodiments, the light detector is a camera. In some embodiments, this may be the CLI camera. An additional detector may be used to prevent damage to the CLI camera if, for example, the level of illumination is too great.

Fig. 5 shows an alternative embodiment. In this embodiment the imaging means comprises two cameras, an emCCD camera for CLI and a colour video camera for illuminated (e.g. anatomical) imaging. This figure also illustrates the white light source at the proximal end of the fibrescope that can be used to illuminate the shrouded region of the patient. An optical coupler and shutter direct light to the two cameras and shutter is emCCD camera during periods when the interior of the shroud is illuminated. The shutter is controlled by a shutter controller to be synchronised with the illumination.

Fig. 6a shows one exemplary configuration for the optical coupler and shutter. In this example a single aspheric lens is used for coupling out of the fibre bundle and focussing on to both cameras. A high transmittance beam splitter option is shown.

Fig. 6b shows another option for the configuration of the optical coupler. In this example, a modular setup is used with a collimating lens coupling out of the fibre bundle and a focussing lens attached to each camera. The high transmittance beam splitter option is shown in this example too.

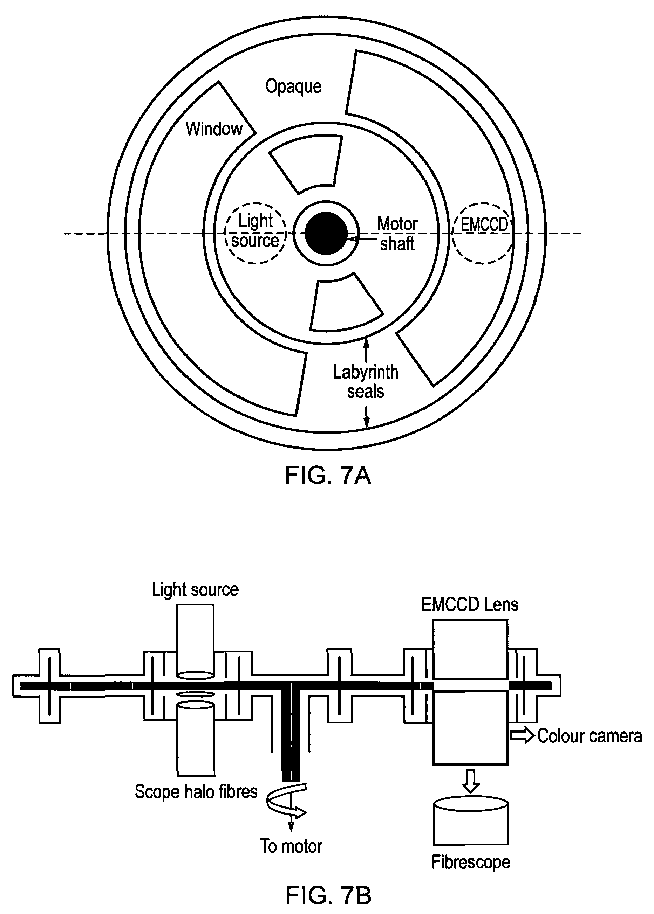

Figs. 7a and 7b show an alternative shutter arrangement that can be used to shutter both the light source and the emCCD camera in embodiments where these two components are located adjacent to one another. The shutter is a rotating disc that is configured to cover both the light source and the emCCD lens. The disc is driven by a motor and as it rotates, windows (holes) in the disc, aligned respectively with the light source and the emCCD lens (which are at different radii) mean that the light source and the emCCD camera lens are selectively covered and uncovered. The relative positions of the windows ensure that the emCCD lens is covered when the light source is uncovered. As best seen in fig. 7a, the emCCD windows are longer than the windows for the light source, to give a longer CLI acquisition period compared with the illuminated image acquisition period.

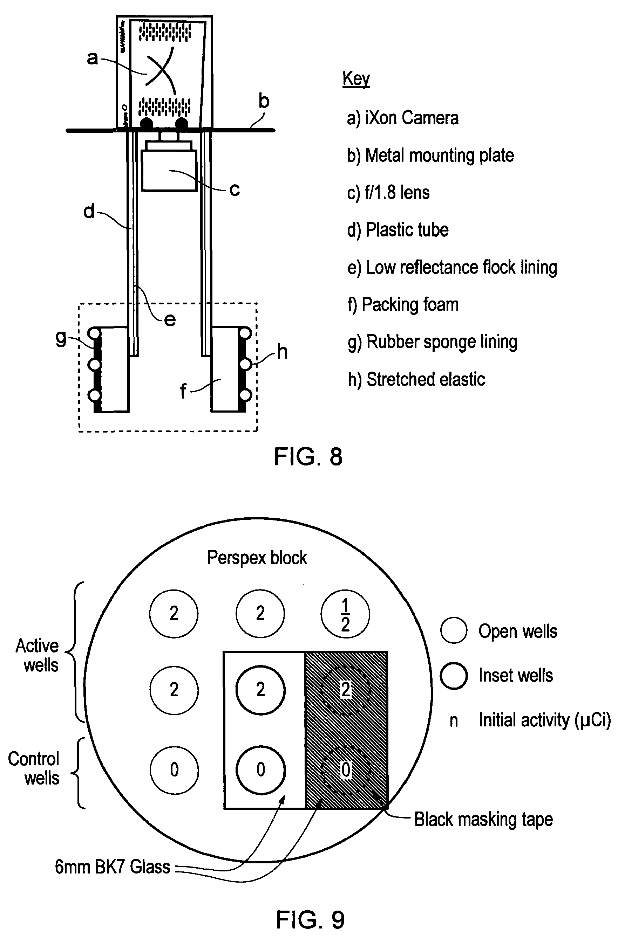

Fig. 8 shows a schematic view of an exemplary optical light shield and camera setup in line with the present invention and used for the experiments discussed below. In this example, an iXon camera is positioned directly above a sample to be imaged (not shown) on a metal mounting plate b. An f/1.8 lens c is located beneath the camera and metal mounting plate b. A plastic (PVC) tube d extends between the metal mounting plate b and the sample to be imaged. The plastic tube d is lined with a low reflectance flock lining e. The sample is surrounded by packing foam g, which is covered with a neoprene rubber sponge lining g, in turn covered with stretched elastic h.

In some embodiments, a phantom, or testing replica, for ultra-weak light may be used to calibrate the light system. The phantom may use a light emitting diode (LED) with a stack or layers of neutral density filters. If necessary, the LED may be driven with a modulated waveform to further and controllably reduce the output of the LED. Such a phantom may also or alternatively be useful for maintenance and quality control of the light system.

The skilled person will appreciate that various modification to the specifically described embodiment are possible without departing from the invention. The following examples are used to support certain aspects of the invention.

Examples

Example 1

An iXon Ultra 897 emCCD camera was used to detect the extremely low light levels expected in CLI. The source was simulated using a duty cycled LED with neutral density filters and a diffuser. An f/2.8 lens was used, though the light levels were scaled to simulate an f/1 lens.

The target irradiance was 0.013 photons/pixel/s.

Calibration

The number of photons produced by the LED was scaled by the duty cycle (proportion of time the LED was on in 1 s) distributed by the collimator into a circular beam with a 25 mm diameter. Once attenuated by neutral density filters it encountered the heavy diffuser. This randomised the direction of each photon resulting in half the photons being emitted at the other side. These photons were now divergent and were spread over 2n sr (a hemisphere). This provided radiance in photons/s/sr/cm$^2$, and allowed prediction of the irradiance of the detector.

| Setup | Filters | Duty Cycle (fraction on) | Magnification | Predicted Irradiance (photons/pixel/s) | | |
|---|---|---|---|---|---|---|
| | | | | Maximum | Nominal | Minimum |
| 1 | OD4 | 1/10 | 0.310 | 1.63 | 1.44 | 1.26 |
| 2 | OD4 | 1/50 | 0.310 | 0.326 | 0.288 | 0.252 |
| 3 | OD4+OD2 | 1/10 | 0.310 | 0.017 | 0.014 | 0.012 |
| 4 | OD4+OD2 | 1/10 | 0.246 | 0.019 | 0.016 | 0.014 |
| 5 | OD4+OD2 | 1/100 | 0.246 | 0.0019 | 0.0016 | 0.0014 |

*Rubber sponge shroud*

For low irradiance measurements, the OD4 and OD2 filters were used with a 1/10 duty cycle. The camera was used in photon counting mode, which digitized each pixel using a photon threshold in counts, removing the impact of the excess noise factor. The camera setup was as follows:

- CCD thermoelectrically cooled to -80 °C
- 1 MHz Gain 3 pre-amplification
- 1000x EM Gain
- 16 x 16 images (32 x 32 binning)
- photon counting threshold = 500
- f/2.8 lens
- 0.5 μs shift speed

The signal for a 1 s exposure with the room lights off was 0.0048 photons/pixel. This corresponded to S:N for a 32 x 32 binned pixel of 1.7.

The lower than expected photon counts could have been attributed to the photon counting threshold used and the small, but (now) significant contribution to the background due to scattered light from source leakage, making the signal appear artificially small.

Turning on room lights led to a 25 x higher background than the signal levels detected from the source, perhaps due to small gaps in the shrouding to surface interface.

Measurements were taken with duty cycles of 1/2 and 1/5.

To summarize, it was possible to measure the target irradiance with the room lights off. The photon counting required very high levels of gains and was not very effective. The rubber sponge shroud was good at blocking light. It was found to fold and create gaps at the interface which, combined with the large perimeter, allowed in too much light to make measurements with room lights on.

*Silicone cone shroud*

OD4 and OD2 filters were used with a 1/10 duty cycle.

- CCD thermoelectrically cooled to -80 °C
- 1 MHz Gain 3 pre-amplification
- 300 x EM Gain
- 16 x 16 images (32 x 32 binning)
- f/2.8 lens
- 0.5 $\mu$s shift speed

Signal for a 1 s exposure with the room lights off was found to be 0.0015 photons/pixel, corresponding to a S:N for a 32 x 32 binned pixel of 2.1.

Turning on background lights led to a signal that did not correspond with the source, due to the scattering of ambient photons. The image of the source was 5 times brighter than the source radiance and so the source could not be measured with the room lights on.

10 times less radiance could be measured with the room lights off (using a duty cycle of 1 to 100). For a 10 second measurement the signal level was 0.019 photons/pixel, giving S:N of 2.1.

To summarize, it was possible to measure the target irradiance with the room lights off. It was possible to measure 10 times lower radiance using a 10 times longer exposure. The silicone cone shroud provided a good quality seal on the flat surface, but was not sufficiently opaque and allowed light to be transmitted into the shielded area.

*Rubber sponge and silicone cone combined shroud*

The OD4 and OD2 filter was used with a 1.10 and 1.100 duty cycle. The camera setup was as follows:

- CCD thermoelectrically coled to - 80 °C
- 1 MHz Gain 3 pre-amplification
- 300x EM Gain or 100x EM Gain
- 16 x 16 images (32 x 32 binning)
- f/2.8 lens
- 0.5 $\mu$s shift speed

The signal for a 1 s exposure with the room lights on was 0.0016 photons/pixel, corresponding to S:N for a 32 x 32 binned pixel of 3.1.

Additional measurements were taken with as low as 10 times less irradiance and 100 x EM Gain. Some of these are shown in the table below.

| Source duty cycle | EM Gain | Integration time(s) | Signal (photons/pixel) | S:N | Magnification |
|---|---|---|---|---|---|
| 1/10 | 100 | 1 | 0.015 | 2.2 | 0.246 |
| 1/10 | 300 | 1 | 0.016 | 3.1 | 0.246 |
| 1/100 | 100 | 10 | 0.017 | 2.4 | 0.246 |
| 1/100 | 300 | 10 | 0.016 | 1.9 | 0.246 |

To summarize, it was possible to measure the target irradiance with the room lights on and 100x EM Gain.

Therefore, it can be concluded that use of an appropriate shroud, such as a silicone cone with rubber sponge layers around the outside, can be used to shield ambient light to such a level that it is possible to employ CLI with room lights on.

<u>Example 2</u>

*In vitro* measurements of Cerenkov radiation emitted from F18 FDG were conducted using an iXon Ultra 897 emCCD camera.

The camera was set up so that the experiment could be conducted inside a lead enclosure with the operation of the laptop on the other side of a room. The camera had the following settings:

- 50 mm f/1.8 lens
- CCD temperature: -80 °C
- 1 MHz pre-amplifier with a gain setting 3
- 0.5 $\mu$s vertical shift speed
- 300x EM Gain
- The field of view is 47x47 mm

F18 was diluted and distributed into six 0.2 mL experimental wells inside a Perspex™ (PMMA) block. Three control wells with inactive material were also prepared. Fig. 7 illustrates the layout of the experimental wells.

The liquid volume and initial activity concentration in the active wells is shown in the table below.

| Activity ($\mu$Ci) | Volume ($\mu$l) | Activity concentration (nCi/$\mu$l) | No. wells |
|---|---|---|---|
| 2 | 200 | 10 | 4 |
| 1 | 100 | 10 | 1 |
| 0.5 | 200 | 2.5 | 1 |

One control well and one active well with activity 2 $\mu$Ci were covered with 6 mm thick BK7 glass. One control well and one active well with activity 2 $\mu$Ci were covered with 6 mm thick BK7 glass and black masking tape. The BK7 glass is inset, with the wells under it 6 mm below the level of the other wells as viewed by the camera. The black masking tape was placed between the wells and the glass, leaving the glass open for viewing.

The sample block was prepared and placed under the shielded camera, which was then lowered into place and draped to give a light tight enclosure. Images with the following settings were acquired:

1. 1s integration time, 16x16 resolution (32x32 binning)
2. 3s integration time, 16x16 resolution (32x32 binning)
3. 5s integration time, 32x32 resolution (16x16 binning)

Further images with the same settings were taken, with the room lights on and off, at regular intervals throughout the experiment.

After the experiment each image was exported and the raw data in counts is converted to a signal in photo-electrons (or detected photons) using the following formula:

$$Signal(photoelectrons) = \frac{(Signal(counts) - Bias\ Offset) \times Conversion\ Factor}{EM\ Gain}$$

Bias Offset = 200 counts
Conversion Factor = 4.27 electrons/count
EM Gain = 300

*Results*

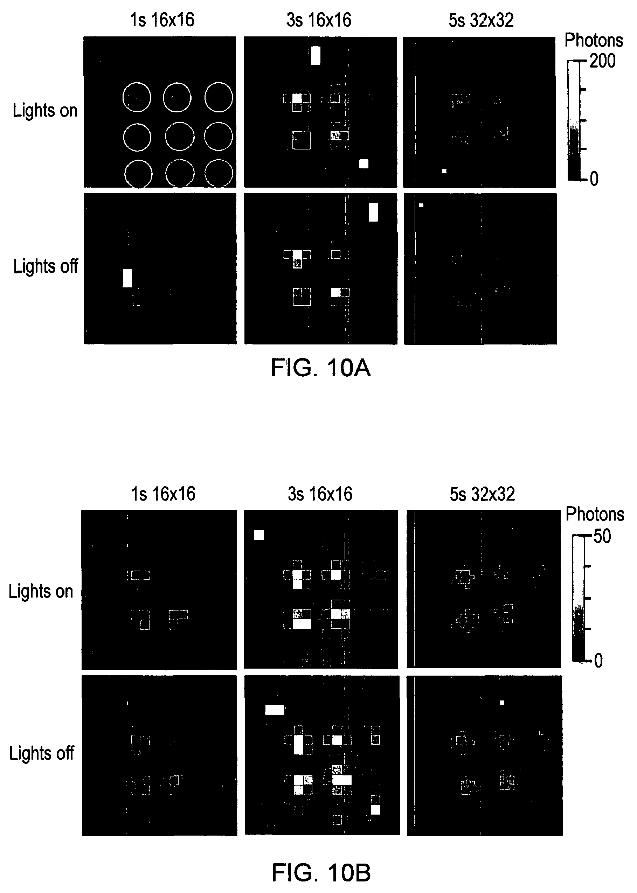

Fig. 10a shows a set of images taken approximately 15 minutes into the experiment with the lights on and lights off. The positions of the wells have been circled in the top left image using the same scheme as figure 6. It can be seen that a higher resolution of 32x32 is obtainable in 5 seconds. In addition to the Cherenkov emission interference from high energy rays can be seen as random white pixels that have a signal level beyond the scale used.

Fig. 10b shows set of images taken approximately 180 minutes into the experiment. Even with the lower signal levels there is no discernible difference due to the room lights. The higher activity wells are still easily visible and the lowest activity well is still discernible.

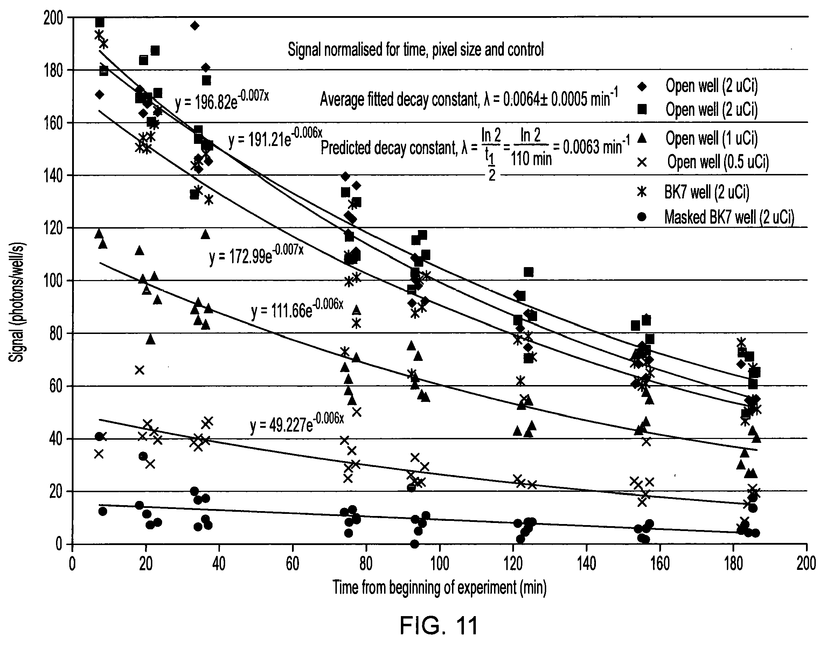

Fig. 11 shows the signal photon rate for each active sample well, after correcting for the background by subtracting the corresponding control signal. Each point on the graph represents an image of the sample. The measured decay constant corresponds to a half-life of 108 minutes.

All three control samples showed no signal. The signal from the open wells was proportional to their initial activity. The signals showed exponential decay with a half-life matching that of the F18 FDG (110 minutes). Therefore, it was concluded that Cerenkov radiation due to activity of F18 FDG was being measured.

The active well covered by glass but not masked produced a similar signal to the open active wells. The masked well showed no visible signal, and was quantified to be 10% of the signal obtained from the unmasked well. Therefore it was concluded that the scintillation in optical BK7 glass was insignificant.

There was significant interference from gamma rays. However, it was shown in principle that higher resolutions are possible if the sensor is shielded from gamma rays and direct particle impingement. Further, it was possible to detect activities as low as 160 nCi (0.8 nCi/$\mu$l) with a spatial resolution down to 400 $\mu$m.

## Claims

1. An apparatus configured for optical imaging of Cerenkov luminescence from a region of interest (18) on a subject (14) subsequent to the subject (14) receiving a dose of a radiopharmaceutical, the apparatus comprising:

   an imaging means (12) capable of imaging Cerenkov photons;
   a fibrescope (10) for transmitting light received at a distal end (16) of the fibrescope (10) to a proximal end of the fibrescope (10), the proximal end of the fibrescope (10) being connected to the imaging means (12); **characterised in that** it further comprises,
   an optical shroud (22) surrounding the distal end (16) of the fibrescope (10) and covering, when in use, said region of interest (18), wherein the optical shroud (22) is capable of at least substantially blocking the passage of ambient fight.

2. An apparatus according to claim 1, further comprising an opaque drape to be placed over the subject, the drape having an opening and the bottom edge of the shroud being configured to engage with the drape around the opening.

3. An apparatus according to claim 2, comprising sealing means to secure the shroud to the drape, wherein the sealing means is a physical, pneumatic, hydraulic, magnetic or electrostatic seal, or a physical connector.

4. An apparatus according to any one of the preceding claims, comprising a light sensor within the shroud.

5. An apparatus according to any one of the preceding claims, wherein the fibrescope (10) comprises a lens at the distal end (16) and adjustment of the lens focus is motorised.

6. An apparatus according to any one of the preceding claims, comprising a radiation shield for the fibre optic bundle within the fibrescope (10) or the complete fibrescope (10).

7. An apparatus according to any one of the preceding claims, comprising an illumination channel in the fibrescope (10) through which light from a light source can be transmitted from the proximal end to the distal end (16) of the fibrescope (10) to provide an illumination source at the tip of the fibrescope (10) within the shroud (22).

8. An apparatus according to any one of claims 1 to 6, comprising one or more light sources mounted within the shroud (22).

9. Apparatus according to claim 7 or claim 8, comprising a mechanical shutter to cover the light source(s) during periods of Cerenkov Luminescence Imaging, CLI, acquisition and to uncover the light source(s) during periods of illuminated image acquisition, wherein the mechanical shutter is synchronised to the image acquisition sequence.

10. Apparatus according to any one of claims 7 to 9, wherein the apparatus is controlled such that duration on Cerenkov Luminescence Imaging, CLI, acquisition periods is longer than illuminated image acquisition periods.

11. An apparatus according to any one of the preceding claims, comprising a Cerenkov radiator that can be placed over the tissue surface at the region of interest (18).

**12.** An apparatus according to any one of the preceding claims, wherein the fibrescope (10) is housed in a sterile casing.

**13.** A method for optical imaging of Cerenkov luminescence from a region of interest (18) on a subject (14) subsequent to the subject (14) receiving a dose of a radiopharmaceutical, the method comprising:

covering the region of interest (18) with a shroud (22) to form a light tight enclosure over the region of interest (18); and
capturing a Cerenkov luminescence image of the region of interest (18) using a fbrescope (10) having a distal end (16) within the shroud (22) and a proximal end outside of the shroud (22) connected to an imaging means (12).

**14.** The method of claim 13, further comprising illuminating the interior of the shroud (22) with light and capturing an illuminated image of the region of interest (18) whilst the interior of the enclosure is illuminated, the Cerenkov luminescence image being captured when the interior of the enclosure is not illuminated.

**15.** The method of claim 13 or claim 14, wherein the method comprises using the apparatus of any one of claims 1 to 12.

**Patentansprüche**

**1.** Vorrichtung, welche zur optischen Abbildung einer Cerenkov-Lumineszenz von einem interessierenden Bereich (18) bei einem Patienten (14) im Anschluss daran ausgebildet ist, dass der Patient (14) eine Dosis eines Radiopharmakons empfangen hat, wobei die Vorrichtung aufweist:

ein Abbildungsmittel (12), welches Cerenkov-Photonen abbilden kann,
ein Fibroskop (10), um das Licht, das an einem distalen Ende (16) des Fibroskops (10) empfangen worden ist, an ein proximales Ende des Fibroskops (10) zu übertragen, wobei das Fibroskop (10) mit dem Abbildungsmittel (12) verbunden ist, **dadurch gekennzeichnet, dass** es weiter aufweist:

eine optische Abschirmung (22), welche das distale Ende des Fibroskops (10) umgibt und welche bei der Benutzung den interessierenden Bereich (18) abdeckt, wobei die optische Abschirmung (22) im Wesentlichen mindestens den Durchgang von Umgebungslicht blockieren kann.

**2.** Vorrichtung nach Anspruch 1, welche ferner ein opakes Abdecktuch aufweist, welches auf den Patienten gelegt werden soll, wobei das Abdecktuch eine Öffnung aufweist und wobei der untere Rand der Abschirmung ausgestaltet ist, mit dem Abdecktuch im Eingriff um die Öffnung zu sein.

**3.** Vorrichtung nach Anspruch 2, welche ferner ein Dichtungsmittel aufweist, um die Abschirmung an dem Abdecktuch zu befestigen, wobei das Dichtungsmittel eine physikalische Dichtung, eine pneumatische Dichtung, eine hydraulische Dichtung, eine magnetische Dichtung oder eine elektrostatische Dichtung oder ein physikalisches Verbindungsmittel ist.

**4.** Vorrichtung nach einem der vorhergehenden Ansprüche, welche einen Lichtsensor innerhalb der Abschirmung aufweist.

**5.** Vorrichtung nach einem der vorhergehenden Ansprüche, wobei das Fibroskop (10) eine Linse an dem distalen Ende (16) aufweist und wobei eine Einstellung des Linsenfokus mit einem Motor versehen ist.

**6.** Vorrichtung nach einem der vorhergehenden Ansprüche, welche einen Strahlungsschild für das Faseroptikbündel innerhalb des Fiberskops (10) oder für das vollständige Fiberskop (10) aufweist.

**7.** Vorrichtung nach einem der vorhergehenden Ansprüche, welche einen Beleuchtungskanal in dem Fibroskop (10) aufweist, durch welchen das Licht von einer Lichtquelle von dem proximalen Ende an das distale Ende (16) des Fiberskops (10) übertragen werden kann, um eine Beleuchtungsquelle an der Spitze des Fiberskops (10) innerhalb der Abschirmung (22) bereit zu stellen.

**8.** Vorrichtung nach einem der Ansprüche 1 bis 6, welche eine oder mehrere Lichtquellen aufweist, welche innerhalb der Abschirmung angebracht sind.

**9.** Vorrichtung nach Anspruch 7 oder 8, welche einen mechanischen Verschluss aufweist, um die Lichtquelle(n) während der Perioden der Abbildung der Cerenkov-Lumineszenz, der CLI und der Erfassung abzudecken und um die Lichtquelle(n) während der Perioden der Erfassung des beleuchteten Bildes aufzudecken, wobei der mechanische Verschluss mit der Bildaufnahmesequenz synchronisiert ist.

**10.** Vorrichtung nach einem der Ansprüche 7 bis 9, wobei die Vorrichtung so gesteuert wird, dass eine Zeitdauer bei den Perioden der Abbildung der Cerenkov-Lumineszenz, der CLI und der Erfassung länger als eine Zeitdauer bei den Perioden der Erfassung des beleuchteten Bildes ist.

**11.** Vorrichtung nach einem der vorhergehenden Ansprüche, welche einen Cerenkov-Heizkörper aufweist, welcher über die Gewebeoberfläche in dem interessierenden Bereich angeordnet werden kann.

**12.** Vorrichtung nach einem der vorhergehenden Ansprüche, wobei das Fibroskop (10) in einem sterilen Gehäuse untergebracht ist.

**13.** Verfahren zur optischen Abbildung einer Cerenkov-Lumineszenz von einem interessierenden Bereich (18) bei einem Patienten (14) im Anschluss daran, dass der Patient (14) eine Dosis eines Radiopharmakons empfangen hat, wobei das Verfahren aufweist:

ein Bedecken des interessierenden Bereiches (18) mit einer Abschirmung (22), um einen lichtdichten Einschluss über den interessierenden Bereich (18) zu erzeugen, und
ein Erfassen eines Bildes der Cerenkov-Lumineszenz des interessierenden Bereiches (18) mit einem Fibroskop (10), welches ein distales Ende (16) innerhalb der Abschirmung (22) und ein proximales Ende, das mit einem Abbildungsmittel (12) verbunden ist, außerhalb der Abschirmung (22) aufweist.

**14.** Verfahren nach Anspruch 13, welches ferner ein Beleuchten des Inneren der Abschirmung (22) mit einem Licht und ein Erfassen des beleuchteten Bildes des interessierenden Bereiches (18) aufweist, während das Innere beleuchtet wird, wobei das Bild der Cerenkov-Lumineszenz erfasst wird, während das Innere des Einschlusses nicht beleuchtet wird.

**15.** Verfahren nach Anspruch 13 oder 14, wobei das Verfahren das Verwenden der Vorrichtung nach einem der Ansprüche 1 bis 12 aufweist.

**Revendications**

**1.** Appareil configuré en vue d'une imagerie optique de luminescence de Cerenkov à partir d'une région d'intérêt (18) sur un sujet (14), après réception, par le sujet (14), d'une dose d'un produit radio-pharmaceutique, l'appareil comprenant :

un moyen d'imagerie (12) en mesure de réaliser l'imagerie de photons de Cerenkov ;
un fibroscope (10) pour transmettre de la lumière reçue au niveau d'une extrémité distale (16) du fibroscope (10) à une extrémité proximale du fibroscope (10), l'extrémité proximale du fibroscope (10) étant connectée au moyen d'imagerie (12) ; **caractérisé en ce qu'**il comprend en outre :

une enveloppe optique (22) entourant l'extrémité distale (16) du fibroscope (10) et recouvrant, en cours d'utilisation, ladite région d'intérêt (18), dans lequel l'enveloppe optique (22) est apte à bloquer au moins sensiblement le passage de la lumière ambiante.

**2.** Appareil selon la revendication 1, comprenant en outre un champ opératoire opaque destiné à être placé sur le sujet, le champ opératoire présentant une ouverture et le bord inférieur de l'enveloppe étant configuré de manière à être en prise avec le champ opératoire autour de l'ouverture.

**3.** Appareil selon la revendication 2, comprenant un moyen d'étanchéité pour fixer l'enveloppe au champ opératoire, dans lequel le moyen d'étanchéité est un joint physique, pneumatique, hydraulique, magnétique ou électrostatique, ou un connecteur physique.

**4.** Appareil selon l'une quelconque des revendications précédentes, comprenant un capteur de lumière au sein de l'enveloppe.

**5.** Appareil selon l'une quelconque des revendications précédentes, dans lequel le fibroscope (10) comprend un objectif au niveau de l'extrémité distale (16) et dans lequel le réglage de la mise au point d'objectif est motorisé.

**6.** Appareil selon l'une quelconque des revendications précédentes, comprenant un écran de protection radiologique pour le faisceau de fibres optiques au sein du fibroscope (10) ou pour le fibroscope complet (10).

**7.** Appareil selon l'une quelconque des revendications précédentes, comprenant un canal d'éclairage dans le fibroscope (10), à travers lequel de la lumière provenant d'une source de lumière peut être transmise, de l'extrémité proximale à l'extrémité distale (16) du fibroscope (10), en vue de fournir une source d'éclairage à la pointe du fibroscope (10) dans l'enveloppe (22).

**8.** Appareil selon l'une quelconque des revendications 1 à 6, comprenant une ou plusieurs sources de lumière montées au sein de l'enveloppe (22).

**9.** Appareil selon la revendication 7 ou 8, comprenant un obturateur mécanique destiné à recouvrir la ou les sources de lumière pendant des périodes d'acquisition d'imagerie de luminescence de Cerenkov, CLI, et à découvrir la ou les sources de lumière pendant des périodes d'acquisition d'image éclairée, dans lequel l'obturateur mécanique est synchronisé sur la séquence d'acquisition d'image.

**10.** Appareil selon l'une quelconque des revendications 7 à 9, dans lequel l'appareil est commandé de sorte qu'une durée sur des périodes d'acquisition d'imagerie de luminescence de Cerenkov CLI est plus longue que sur des périodes d'acquisition d'image éclairée.

**11.** Appareil selon l'une quelconque des revendications précédentes, comprenant un radiateur de Cerenkov qui peut être placé au-dessus de la surface tissulaire au niveau de la région d'intérêt (18).

**12.** Appareil selon l'une quelconque des revendications précédentes, dans lequel le fibroscope (10) est logé dans un boîtier stérile.

**13.** Procédé d'imagerie optique de luminescence de Cerenkov d'une région d'intérêt (18) sur un sujet (14), après réception, par le sujet (14), d'une dose d'un produit radio-pharmaceutique, le procédé comprenant les étapes ci-dessous consistant à :

recouvrir la région d'intérêt (18) d'une enveloppe (22) en vue de former une enceinte étanche à la lumière sur la région d'intérêt (18) ; et
capturer une image de luminescence de Cerenkov de la région d'intérêt (18) à l'aide d'un fibroscope (10) présentant une extrémité distale (16) au sein de l'enveloppe (22) et une extrémité proximale à l'extérieur de l'enveloppe (22), connecté à un moyen d'imagerie (12).

**14.** Procédé selon la revendication 13, comprenant en outre l'étape consistant à éclairer l'intérieur de l'enveloppe (22) avec de la lumière et à capturer une image éclairée de la région d'intérêt (18), tandis que l'intérieur de l'enceinte est éclairé, l'image de luminescence de Cerenkov étant capturée lorsque l'intérieur de l'enceinte n'est pas éclairé.

**15.** Procédé selon la revendication 13 ou 14, dans lequel le procédé comprend l'utilisation de l'appareil selon l'une quelconque des revendications 1 à 12.

S = Subject, LS = Blue-light source, BS = Beam splitter, BP = Band-pass filter, C1 = Camera1 (for illuminated image), C2 = Camera2 (for Cerenkov image), 1 = Image1, I2 = Image2, P = Image processing, RS = Radiation shielding, I = Final image

FIG. 1

S = Subject, LS = Blue-light source, BS = Beam splitter, DMD = Digital Micromirror
Device BP = Band-pass filter, C1 = Camera1 (for illuminated image), C2 = Camera2 (for
Cerenkov image), 1 = Image1, I2 = Image2, P = Image processing, RS = Radiation
shielding, I = Final image, TR = Trigger, L1, L2, L3 = Lenses

FIG. 2

PD = Pulse duration, PI = Pulse interval, GO = Gating offset

FIG. 3

20

10

12

22    16    24

18    14

FIG. 4A

FIBRESCOPE

FABRIC GASKET
ALLOWS MOVEMENT

LIGHT TIGHT
SEAL

RIGID RING

SPONGE

DRAPE

SURGICAL
SITE

TISSUE

FIG. 4B

LIGHT TIGHT
PORT IN
INCISION

ENDOSCOPE

COUPLING BETWEEN
DRAPES, VELCRO,
MAGNETIC,
ELECTROSTATIC ETC

2nd DRAPE

PRIMARY
DRAPE OVER
PATIENT

TISSUE

FIG. 4C

SCOPE

SKIRT

SPONGE

DRAPE

TISSUE

FIG. 4D

FIG. 5

FIG. 6A                    FIG. 6B

FIG. 7A

FIG. 7B

Key

a) iXon Camera

b) Metal mounting plate

c) f/1.8 lens

d) Plastic tube

e) Low reflectance flock lining

f) Packing foam

g) Rubber sponge lining

h) Stretched elastic

FIG. 8

FIG. 9

FIG. 10A

FIG. 10B

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110250128 A
- US 20120220870 A
- WO 2012083503 A
- WO 2011137247 A

**Non-patent literature cited in the description**

- **ROBERTSON et al.** *Phys Med Biol.,* 2009
- **HOLLAND et al.** *Mol Imaging,* 2011
- **CARPENTER et al.** *J Nucl. Med,* 2012
- **KOTHAPALLI et al.** *Biomedical Optics Express,* 01 June 2012, vol. 3 (6
- **ZHONG et al.** *International Journal of Biomedical Imaging,* vol. 2011
- Endoscopic imaging of Cerenkov Luminescence. *Biomedical Express,* 03 May 2012, vol. 3 (6